# FASCICULE DE BREVET EUROPEEN

(11) **EP 0 854 711 B1**
(45) Date de publication et mention de la délivrance du brevet: **23.01.2002**
(21) Numéro de dépôt: 96933503.3
(22) Date de dépôt: 08.10.1996
(51) Int. Cl.: A61K 31/19, A61K 31/455, A61P 43/00

(54) **LIGANT ANTAGONISTE RAR-gamma OU AGONISTE RAR-alpha EN TANT QU'INHIBITEUR D'APOPTOSE**
gamma-RAR ANTAGONISTLIGAND ODER alpha-RAR AGONISTLIGAND ALS APOPTOSEINHIBITOR
gamma-RAR ANTAGONIST LIGAND OR alpha-RAR AGONIST LIGAND AS AN APOPTOSIS INHIBITOR

(30) Priorité: 11.10.1995 FR 9511946
(43) Date de publication de la demande: 29.07.1998
(73) Titulaire: CENTRE INTERNATIONAL DE RECHERCHES DERMATOLOGIQUES GALDERMA (C.I.R.D. GALDERMA), 06560 Valbonne (FR)
(72) Inventeur: FESUS, Laszlo, H-4032 Debrecen (HU); SZONDY, Zsuzsa, H-4032 Debrecen (HU); REICHERT, Uwe, F-06620 Pont-du-Loup (FR)
(74) Mandataire: Tezier Herman, Béatrice
(86) Numéro de dépôt international: FR9601569
(87) Numéro de publication internationale: WO9713506

(56) Documents cités:
- EP-A- 0 658 553
- J.BIOL. CHEM., vol. 270, no. 11, 1995, pages 6022-6029, XP000574946 L. ZHANG ET AL.: "Evidence for the involvement of retinoic acid receptor RAR alpha-dependent signalling pathway in the induction of tissue transglutaminase and apoptosis by retinoids."
- LEUKEMIA, vol. 8, no. SPL.3, 1994, pages S83-S84, XP000575616 P.S. GILL ET AL.: "Solid tumor treatment workshop summary."

## Description

La présente invention concerne l'utilisation de rétinoïdes particuliers dans la préparation d'une composition pharmaceutique en vue de diminuer le taux d'apoptose.

Il existe deux types de mécanisme impliqués dans la mort des cellules. Le premier de type classique est appelé la nécrose. Morphologiquement, la nécrose est caractérisée par un gonflement des mitochondries et du cytoplasme et par une altération nucléaire, suivi de la destruction de la cellule et son autolyse, ceci étant accompagné par un phénomène d'inflammation. La nécrose survient de manière passive et incidente. La nécrose tissulaire est généralement due à un trauma physique des cellules ou un poison chimique, par exemple.

L'autre forme de mort cellulaire est appelée l'apoptose [Kerr, J.F.R. and Wyllie, A.H., Br. J. Cancer, 265, 239 (1972)], mais contrairement à la nécrose l'apoptose n'entraîne pas un phénomène d'inflammation. Il est décrit que l'apoptose peut se réaliser sous différentes conditions physiologiques. C'est une forme hautement sélective du suicide cellulaire qui se caractérise par des phénomènes morphologiques et biochimiques aisément observables. Ainsi, on observe notamment une condensation de la chromatine associée ou non à une activité endonucléasique, la formation de corps apoptiques et une fragmentation de l'acide désoxyribonucléique (A.D.N.) par l'activation d'endonucléases en des fragments d'A.D.N. de 180-200 paires de bases (ces fragments peuvent être observés par électrophorèse sur gel d'agarose).

L'apoptose peut être considérée comme une mort programmée des cellules impliquée dans le développement, la différenciation et le renouvellement tissulaire. Il est également considéré que la différenciation, la croissance et la maturation des cellules sont étroitement liées à l'apoptose et que les substances capables de jouer un rôle sur la différenciation, la croissance et la maturation des cellules sont aussi liées au phénomène de l'apoptose. Ainsi, chez un être humain en bonne santé, il existe un équilibre entre l'ensemble de ces phénomènes.

Dans le domaine médical, un certain nombre de situations pathologiques présente un mécanisme d'apoptose modifié, voire déréglé, ou un mécanisme d'apoptose qui ne pourvoit pas à un dérèglement d'un autre phénomène biologique pour arriver à l'équilibre. Ainsi, il est décrit qu'une modulation volontaire de l'apoptose en l'induisant ou en la réprimant peut permettre de traiter de nombreuses maladies, telles que des maladies liées à une insuffisance du taux d'apoptose, telles que dans le cas du cancer, des maladies auto-immunes ou des allergies, ou au contraire des maladies liées à un excès du taux d'apoptose, telle que dans les cas du syndrome d'immunodéficience du virus d'immunodéficience humain (H.I.V.), des maladies neuro-dégénératives (maladie d'Alzheimer) ou des dommages excessifs induits lors de l'infarctus du myocarde.

De manière concrète, il a déjà été décrit de nombreux inhibiteurs d'apoptose, tels que la cycloheximide, la cyclosporine et certaines interleukines.

Dans le domaine des rétinoïdes, on sait que l'acide rétinoïque *tout-trans* est un puissant modulateur (i.e. un inhibiteur ou, au contraire, un stimulateur, selon la nature des cellules traitées) de la différenciation et de la prolifération de nombreux types cellulaires normaux ou transformés. Par exemple, il inhibe la différenciation des cellules épithéliales, tels que les kératinocytes de l'épiderme. Il inhibe aussi la prolifération de nombreuses cellules transformées telles que les cellules de mélanomes. Ces effets sur la prolifération et la différenciation peuvent affecter simultanément un même type cellulaire, comme cela est par exemple le cas pour les cellules promyélocytaires humaines, référencées HL-60 ; ainsi, il est connu que la prolifération de ces cellules est inhibée par l'acide rétinoïque *tout-trans* et, dans le même temps, que leur différenciation en granulocytes et leur apoptose sont induites.

On sait, d'une manière générale, que l'acide rétinoïque *tout-trans* (all-*trans* retinoic acid) agit sur la différenciation et la prolifération des cellules en interagissant avec des récepteurs nucléaires appelés RARs (Retinoic Acid Receptors) contenus dans le noyau cellulaire. Il existe, à ce jour, trois sous-types identifiés de récepteurs RAR appelés respectivement RAR-α, RAR-β et RAR-γ. Ces récepteurs, aprés fixation du ligand (i.e. de l'acide rétinoïque *tout-trans*), interagissent avec la région promotrice de gènes régulés par l'acide rétinoïque au niveau d'éléments de réponses spécifiques (RARE). Pour se fixer sur les éléments de réponse, les RARs s'hétérodimèrisent avec un autre type de récepteurs connus sous le nom de RXRs. Le ligand naturel des RXRs est l'acide 9-*cis*-rétinoïque. Les RXRs sont considérés comme des "master regulatory proteins" car ils interagissent avec d'autres membres de la superfamille des récepteurs stéroïdiens/thyroïdiens pour former des hétérodimères, comme avec les RARs, tels que le récepteur de la vitamine D3 (VDR), le récepteur de la triiodothyroxine (TR) et les PPARs (Peroxisome Proliferator Activated Receptors). De plus, les RXRs peuvent interagir avec des éléments de réponse spécifiques (RXRE) sous forme d'homodimères. Ces interations complexes et l'existence de multiples récepteurs RARs et RXRs exprimés de manière différente en fonction du tissus et du type cellulaire expliquent les effets pléiotropiques des rétinoïdes dans pratiquement toutes les cellules.

De nombreux analogues structuraux synthétiques de l'acide rétinoïque *tout-trans* ou de l'acide 9-*cis*-rétinoïque, couramment dénommés "rétinoïdes", ont été décrits à ce jour dans la littérature. Certaines de ces molécules sont capables de se fixer et d'activer spécifiquement les RARs ou, au contraire, les RXRs. De plus, certains analogues peuvent se fixer et activer un sous-type de récepteur RAR (α, β ou y) particulier. D'autres analogues, enfin, ne présentent aucune activité sélective particulière vis-à-vis de ces différents récepteurs. A cet égard, et par exemple, l'acide 9-*cis* rétinoïque active à la fois les RARs et les RXRs, sans sélectivité notable pour l'un ou l'autre de ces récepteurs (ligand agoniste non spécifique), alors que l'acide rétinoïque *tout-trans* active, quant à lui, sélectivement les RARs (ligand agoniste spécifique RARs), tous sous-types confondus. D'une manière générale et qualitativement, une substance donnée (ou ligand) est dite spécifique d'une famille de récepteurs donnée (respectivement vis-à-vis d'un récepteur particulier de cette famille) lorsque ladite substance présente une affinité pour l'ensemble des récepteurs de cette famille (respectivement pour le récepteur particulier de cette famille) plus forte que celle qu'elle présente par ailleurs pour tous les récepteurs de toute autre famille (respectivement pour tous les autres récepteurs, de cette même famille ou pas).

Il est décrit que l'acide 9-*cis* rétinoïque et l'acide rétinoïque *tout-trans* sont des modulateurs de l'apoptose (activateur ou inhibiteur de l'apoptose notamment en fonction du type cellulaire) et que l'acide 9-*cis* rétinoïque est le plus actif de ces deux modulateurs, ceci pouvant s'expliquer par le fait qu'il active à la fois les RARs et les RXRs contrairement à l'acide rétinoïque *tout-trans* qui n'active que les RARs.

Compte-tenu de qui a été dit précédemment, il apparaît intéressant de trouver de nouveaux modulateurs de l'apoptose.

Dans ce sens, la Demanderesse vient de découvrir que les ligands agonistes spécifiques aux récepteurs de type RAR-α ou les ligands antagonistes spécifiques aux récepteurs de type RAR-γ sont d'excellents inhibiteurs de l'apoptose dans différents types cellulaires, et plus particulièrement dans les thymocytes, cette apoptose ayant été induite par le biais des récepteurs de type RAR-γ ou des récepteurs des cellules T (T-cell receptors).

Ainsi, la présente invention a pour objet l'utilisation d'au moins un ligand choisi parmi un ligand agoniste spécifique aux récepteurs de type RAR-α et un ligand antagoniste spécifique aux récepteurs de type RAR-γ dans la préparation d'une composition pharmaceutique destinée à diminuer le taux d'apoptose dans au moins une population cellulaire. Plus particulièrement, la population cellulaire correspond à des cellules dans lesquelles l'apoptose peut être régulée par induction et/ou inhibition par le biais des récepteurs de types RAR-γ et/ou RAR-α ou dans laquelle l'apoptose peut être régulée par induction par le biais des récepteurs des cellules T.

La composition pharmaceutique selon l'invention comprend un milieu physiologiquement acceptable.

Par ligand agoniste spécifique aux récepteurs de type RAR-α, on entend selon l'invention tout ligand agoniste présentant une constante de dissociation pour les récepteurs de type RAR-α au moins 10 fois inférieure à sa constante de dissociation pour les récepteurs de type RAR-γ et induisant la différenciation des cellules F9.

Par ligand antagoniste spécifique aux récepteurs de type RAR-γ, on entend selon l'invention tout ligand présentant une constante de dissociation pour les récepteurs de type RAR-γ au moins 10 fois inférieure à sa constante de dissociation pour les récepteurs de type RAR-α et n'induisant pas la différenciation des cellules F9.

On sait en effet que l'acide rétinoïque *tout-trans* et certains de ses analogues sont capables d'induire la différenciation des cellules (F9) de tératocarcinome embryonnaire de souris, on les considère alors comme des agonistes aux récepteurs RARs. La sécrétion de l'activateur plasminogène qui accompagne cette différenciation est un indice de la réponse biologique des cellules F9 aux rétinoïdes (Skin pharmacol. 1990; 3 : pp.256-267).

Les constantes de dissociation sont déterminées au moyen de tests classiques pour l'homme de l'art. Ces tests sont notamment décrits dans les références suivantes : (1) "Selective Synthetic Ligands for Nuclear Retinoic Acid Receptor Subtypes" *in* RETINOIDS, Progress in Research and Clinical Applications, Chapitre 19 (pp 261-267), Marcel Dekker Inc, édité par Maria A. Livrea et Lester Packer ; (2) "Synthetic Retinoids : Receptor Selectivity and Biological Activity" *in* Pharmacol Skin, Basel, Karger, 1993, Volume. 5, pp 117-127 ; (3) "Selective Synthetic Ligands for Human Nuclear Retinoic Acid Receptors" *in* Skin Pharmacology, 1992, Vol. 5, pp 57-65 ; (4) "Identification of Synthetic Retinoids with Selectivity for Human Nuclear Retinoic Acid Receptor-γ" *in* Biochemical and Biophysical Research Communications, Vol. 186, N° 2, Juillet 1992, pp 977-983 ; (5) "Selective High Affinity RAR-α or RAR-β Retinoic Acid Receptor Ligands" *in* Mol. Pharmacol., Vol 40, pp 556-562.

D'autres caractéristiques, aspects, objets et avantages de l'invention apparaîtront encore plus clairement à la lecture de la description qui va suivre, ainsi que des divers exemples concrets, mais nullement limitatifs, destinés à l'illustrer.

Parmi les ligands agonistes spécifiques aux récepteurs de type RAR-α, on peut notamment citer l'acide 4-((5,6,7,8-tetrahydro-5,5,8,8-tetramethyl-2-naphtalenyl)carboxamido) benzoïque, l'acide 4-((5,6,7,8-tetrahydro-5,5,8,8-tetramethyl-2-naphtalenyl)carbamoyl) benzoïque et l'acide 2-hydroxy-4-(5,5,8,8-tetramethyl-5,6,7,8-tetrahydro-naphtalene-2-carboxamido)-benzoique.

De préférence, on utilise dans la présente invention, parmi les ligands agonistes spécifiques aux récepteurs de type RAR-α, au moins un ligand agoniste présentant une constante de dissociation pour les récepteurs de type RAR-α au moins 20 fois inférieure à sa constante de dissociation pour les récepteurs de type RAR-γ, tel que l'acide 4-((5,6,7,8-tetrahydro-5,5,8,8-tetramethyl-2-naphtalenyl)carboxamido) benzoïque et l'acide 2-hydroxy-4-(5,5,8,8-tetramethyl-5,6,7,8-tetrahydro-naphtalene-2-carboxamido)-benzoique.

Parmi les ligands antagonistes spécifiques aux récepteurs de type RAR-γ, on peut notamment citer l'acide 2-hydroxy-4-[7-(1-adamantyl)-6-benzyloxy-2-naphtyl]benzoïque, l'acide 2-hydroxy-4-[7-(1-adamantyl)-6-hexyloxy-2-naphtyl]benzoïque, l'acide 4-[7-(1- 1-adamantyl)-6-méthoxyéthoxyméthoxy-2-naphtyl]benzoïque, l'acide 5-[7-(1-adamantyl)-6-benzyloxy-2-naphtyl]-2-thiophènecarboxylique, l'acide 4-[7-(1-adamantyl)-6-benzyloxy-2-naphtyl]benzoïque, l'acide 4-[7-(1-adamantyl)-6-benzyloxycarbonyl-2-naphtyl]benzoïque, l'acide 2-hydroxy-4-[7-(1-adamantyl)-6-(4-fluorobenzyl)oxy-2-naphtyl] benzoïque, l'acide 4-[7-(1-adamantyl)-6-heptyloxy-2-naphtyl]benzoïque, l'acide 6-[7-(1-adamantyl)-6-méthoxyéthoxyméthoxy-2-naphtyl]nicotinique, l'acide 2-hydroxy-4-[7-(1-adamantyl)-6-méthoxyéthoxyméthoxy-2-naphtyl]benzoïque, l'acide 2-chloro-4-[7-(1-adamantyl)-6-méthoxyéthoxyméthoxy-2-naphtyl]benzoïque, l'acide 4-[7-(1-adamantyl)-6-méthoxybutyloxy-2-naphtyl]benzoïque, l'acide 4-[7-(1-adamantyl)-6-méthoxyméthoxypropyl-2-naphtyl]benzoïque, l'acide 4-[7-(1-adamantyl)-6-méthoxyéthoxypropyl-2-naphtyl]benzoïque et l'acide 4-[7-(1-adamantyl)-6-acétyloxybutoxy-2-naphtyl]benzoïque.

De préférence, on utilise dans la présente invention, parmi les ligands antagonistes spécifiques aux récepteurs de type RAR-γ, au moins un ligand antagoniste présentant une constante de dissociation pour les récepteurs de type RAR-γ au moins 20 fois inférieure à sa constante de dissociation pour les récepteurs de type RAR-α, tel que l'acide 4-[7-(1-adamantyl)-6-méthoxyéthoxyméthoxy-2-naphtyl]benzoïque.

Ainsi, la composition pharmaceutique selon l'invention va pouvoir être utilisée lorsque une diminution du taux d'apoptose est nécessaire pour au moins une population cellulaire. Plus particulièrement, la population cellulaire correspond à des cellules dans laquelle l'apoptose peut être régulée par induction et/ou inhibition par le biais des récepteurs de types RAR-γ et/ou RAR-α ou dans laquelle l'apoptose peut être régulée par induction par le biais des récepteurs des cellules T, et donc notamment dans des populations cellulaires où des récepteurs de type RAR-γ et/ou RAR-α ou des récepteurs des cellules T sont présents, tels que par exemple dans les cellules venant du thymus où ces trois types de récepteurs sont présents.

Une diminution du taux d'apoptose peut s'avérer nécessaire lorsqu'il y a donc un excès du taux d'apoptose, cet excès résultant de conditions génétiques ou acquises présentées par la personne à qui l'on veut administrer la composition pharmaceutique. Ces conditions génétiques ou acquises favorisent l'accumulation de signaux qui induisent l'apoptose ou diminuent le seuil auquel ces signaux induisent l'apoptose.

Parmi les maladies ou les désordres liés à un excès du taux d'apoptose, on peut citer plus particulièrement le syndrome d'immunodéficience du virus d'immunodéficience humain (H.I.V.), les maladies neuro-dégénératives, les syndromes myélodisplasiques (tels que l'anémie aplasique), les syndromes d'ischémie (tels que l'infarctus du myocarde), les maladies du foie induites par des toxines (telles que l'alcool), l'alopécie, les dommages de la peau dus aux U.V., le lichen plan, l'atrophie de la peau, la cataracte ou encore le rejet de greffes.

Ainsi, dans les maladies neuro-dégénératives, on peut citer plus particulièrement la maladie d'Azheimer, la maladie de Parkinson, la sclérose latérale amyotropique et d'autres maladies liées à une dégénération du cerveau, telles que la maladie de Creutzfeld-Jakob.

L'administration de la composition selon l'invention peut être effectuée par voie entérale, parentérale, topique ou oculaire. De préférence, la composition pharmaceutique est conditionnée sous une forme convenant à une application par voie systémique (pour injection ou perfusion).

Par voie entérale, la composition, plus particulièrement la composition pharmaceutique, peut se présenter sous formes de comprimés, de gélules, de dragées, de sirops, de suspensions, de solutions, de poudres, de granulés, d'émulsions, de microsphères ou nanosphères ou vésicules lipidiques ou polymériques permettant une libération contrôlée. Par voie parentérale, la composition peut se présenter sous forme de solutions ou suspensions pour perfusion ou pour injection.

Les ligands choisis parmi les ligands agonistes spécifiques aux récepteurs de type RAR-α et les ligands antagonistes spécifiques aux récepteurs de type RAR-γ utilisés selon l'invention sont généralement administrés à une dose journalière d'environ 0,01 mg/kg à 100 mg/kg en poids corporel en 1 à 3 prises.

Par voie topique, la composition pharmaceutique selon l'invention est plus particulièrement destinée au traitement de la peau et des muqueuses et peut se présenter sous forme d'onguents, de crèmes, de laits, de pommades, de poudres, de tampons imbibés, de solutions, de gels, de sprays, de lotions ou de suspensions. Elle peut également se présenter sous forme de microsphères ou nanosphères ou vésicules lipidiques ou polymériques ou de patches polymériques et d'hydrogels permettant une libération contrôlée. Cette composition par voie topique peut se présenter soit sous forme anhydre, soit sous forme aqueuse.

Par voie oculaire, ce sont principalement des collyres.

Les ligands choisis parmi les ligands agonistes spécifiques aux récepteurs de type RAR-α et les ligands antagonistes spécifiques aux récepteurs de type RAR-γ sont utilisés par voie topique ou oculaire à une concentration généralement comprise entre 0,001 % et 10 % en poids, de préférence entre 0,1 et 1 % en poids, par rapport au poids total de la composition.

Les compositions telles que décrites précédemment peuvent bien entendu en outre contenir des additifs inertes ou même pharmacodynamiquement actifs ou des combinaisons de ces additifs, et notamment : des agents mouillants; des agents dépigmentants tels que l'hydroquinone, l'acide azélaïque, l'acide caféïque ou l'acide kojique; des émollients; des agents hydratants comme le glycérol, le PEG 400, la thiamorpholinone, et ses dérivés ou bien encore l'urée; des agents antiséborrhéiques ou antiacnéiques, tels que la S-carboxyméthylcystéine, la S-benzyl-cystéamine, leurs sels ou leurs dérivés, ou le péroxyde de benzoyle; des agents antifongiques tels que le kétoconazole ou les polyméthylène-4,5 isothiazolidones-3; des antibactériens, des caroténoïdes et, notamment, le β-carotène; des agents anti-psoriatiques tels que l'anthraline et ses dérivés; et enfin les acides eicosa-5,8,11,14-tétraynoïque et eicosa-5,8,11-trynoïque, leurs esters et amides.

Ces compositions peuvent également contenir des agents d'amélioration de la saveur, des agents conservateurs tels que les esters de l'acide parahydroxybenzoïque, les agents stabilisants, des agents régulateurs d'humidité, des agents régulateurs de pH, des agents modificateurs de pression osmotique, des agents émulsionnants, des filtres UV-A et UV-B, des antioxydants, tels que l'α-tocophérol, le butylhydroxyanisole ou le butylhydroxytoluène.

Bien entendu, l'homme du métier veillera à choisir le ou les éventuels composés à ajouter à ces compositions de telle manière que les propriétés avantageuses attachées intrinsèquement à la présente invention ne soient pas ou substantiellement pas altérées par l'addition envisagée.

On va maintenant donner, à titre nullement limitatif, plusieurs exemples destinés à illustrer la présente invention.

### EXEMPLE 1

Cet exemple met en évidence l'efficacité in vitro en tant qu'inhibiteur d'apoptose de ligand antagoniste spécifique de RARγ, l'apoptose ayant été induite par d'autres rétinoïdes.

### Culture et préparation des cellules

Des suspensions de thymocytes sont préparées à partir des glandes de thymus des souris mâles NMRI de 4 semaines (vendus par la société LATI, Gödöllo, Hongrie) non traités. Le milieu utilisé est le milieu RPMI 1640 de Sigma supplémenté en sérum de veau fétal de Gibco, 2mM de glutamine, 100UI de pénicillline et 100 µg de streptomycine/ml. Les thymocytes sont ensuite lavées et diluées pour obtenir une concentration finale de 10⁷ cellules/ml, avant incubation à 37°C dans un incubateur humidifié sous un atmosphère de 5% de CO₂ et 95% d'air. La mort des cellules est mesurée par la prise de bleu de trypan.

### Analyse qualitative et quantitative de l'ADN

Les thymocytes sont incubées dans 24 puits avec divers composés à tester. Après 6 heures d'incubation, 0,8ml de suspensions cellulaires ont été lysées par addition de 0,7 ml de tampon de lyse contenant 0,5% (v/v) de Triton X-100, 10mM Tris, 20mM EDTA, pH 8,0, avant centrifugation pendant 15 minutes à 13000g.
- Analyse quantitative de l'ADN : La teneur en ADN dans le surnageant (les fragments) et le culot (chromatine intacte) a été précipitée avec une quantité équivalente de 10% d'acide trichloroacétique, resuspendue dans 5% d'acide trichloroacétique, puis quantifiée en utilisant le réactif diphénylamine (Burton, K. (1956) Biochem. J.. 62, 315-322).
- Analyse qualitative de l'ADN : En parallèle, le surnageant a été précipité pendant une nuit dans de l'éthanol contenant 0,15mM de NaCI. Les culots sont redissouts dans un tampon contenant 10mM Tris, 1mM d'EDTA, pH 8,0, traités à la RNase, séquentiellement extraits à volume égal de phénol, chloroforme/isoamylalcool (24/1) et précipités dans de l'éthanol avant électrophorèse pendant 3 heures à 60V dans 1,8% de gel d'agarose. Les fragments d'ADN ont ensuite été visualisés par UV après avoir coloré le gel avec du bromure d'éthidium. Les gels obtenus présentent l'image d'une échelle de fragments d'ADN multiples de 180 à 200 paires de bases typiques d'une induction d'apoptose. Le degré de fragmentation est corrélé avec le nombre de cellules mortes positives selon le test au bleu de trypan tout le long des expériences.

Les résultats de l'analyse quantitative sont rassemblés dans le tableau 1 suivant.

**Tableau 1**

| composés | Kd RARα | Kd RARγ | quantités (nM) | % de fragments d'ADN |
|---|---|---|---|---|
| ATRA | 15,5 | 3 | 5000 | 17 |
| 9-cisRA | 7 | 17 | 1000 | 19 |
| CD437 | 6500 | 77 | 300 | 24 |
| CD2665 | >2253 | 110 | 200 | 0,5 |
| CD2665 + ATRA | - | - | 200 5000 | 0 |
| CD2665 + 9-cisRA | - | - | 200 1000 | 1,5 |
| CD2665 + CD437 | - | - | 200 300 | 1,0 |

ATRA est l'acide rétinoïque *tout-trans*
9-cisRA est l'acide 9-cis rétinoïque
CD437 est l'acide 6-3-(1-Adamantyl)-4-hydroxyphényl)-2-naphtanoïque,
CD2665 est l'acide 4-[7-(1-adamantyl)-6-méthoxyéthoxyméthoxy-2-naphtyl]benzoïque

Le pourcentage de fragment d'ADN dans ce tableau correspond à la différence de pourcentage de fragment d'ADN obtenu dans des thymocytes traitées et du pourcentage de fragment d'ADN obtenu dans des thymocytés non traitées (taux basal d'apoptose de ces thymocytes).

Ce tableau montre clairement que l'apoptose induite par ATRA, 9-cisRA et CD437 est inhibée par le CD2665 qui est un ligand antagoniste spécifique des récepteurs de type RAR-γ.

### EXEMPLE 2

Cet exemple met en évidence l'efficacité in vitro en tant qu'inhibiteur d'apoptose de ligand agoniste spécifique de RAR-α, l'apoptose ayant été induite par un autre rétinoïde

On procède de la même manière que dans l'exemple précédent, en modifiant la nature des ligands à tester et leur concentration.

Ainsi, le CD437 (acide 6-3-(1-Adamantyl)-4-hydroxyphényl)-2-naphtanoïque) (à une seule concentration) est mis à incuber avec les thymocytes en présence de différentes concentrations de CD336 (acide 4-((5,6,7,8-tetrahydro-5,5,8,8-tetramethyl-2-naphtalenyl)carboxamido) benzoïque).

Le tableau 2 suivant rassemble les résultats obtenus.

**Tableau 2**

| PRODUITS | QUANTITES (M) | % de fragments d'ADN |
|---|---|---|
| CD437 seul | 0,3.10⁻⁶ | 17 |
| CD336 seul | 10⁻⁸ | 0 |
| | 10⁻⁷ | 0 |
| | 10⁻⁶ | 0 |
| CD437 + CD336 | CD336: 10⁻⁸ | 17 |
| | CD336 : 10⁻⁷ | 5 |
| | CD336: 10⁻⁶ | 0 |

Le pourcentage de fragment d'ADN dans ce tableau correspond à la différence de pourcentage de fragment d'ADN obtenu dans des thymocytes traitées et du pourcentage de fragment d'ADN obtenu dans des thymocytés non traitées (taux basal d'apoptose de ces thymocytes).

Ainsi, ce tableau montre clairement que l'apoptose induite par le CD437 qui est un ligand agoniste spécifique des récepteurs de type RAR-γ est inhibée de manière dose-dépendante par le CD336 qui est un ligand agoniste spécifique des récepteurs de type RAR-α.

### EXEMPLE 3

Cet exemple met en évidence l'efficacité in vitro en tant qu'inhibiteur d'apoptose de ligand agoniste spécifique de RAR-α, l'apoptose ayant été induite par l'activation d'un récepteur des cellulles T

On procède de la même manière que dans l'exemple 1, en modifiant la nature des composés à tester et leur concentration.

Il est connu que les cellules T se différencient en lymphocyes T matures dans le thymus. Pendant cette différenciation, les cellules T prolifèrent et génèrent des récepteurs aux cellules T. Les cellules qui expriment des récepteurs de cellules potentiellement autoréactifs et qui interagissent avec les cellules présentant un antigène subissent l'apoptose(sélection négative) (Smith et al. (1989) Nature 337, 181-184). In vitro, cette sélection peut être mimée en stimulant la molécule CD3 associée avec un récepteur aux cellules T en ajoutant simultanément du dibutyrate de phorbol et un Ca++ ionophore (Iseki et al. (1991) J. Immunol. 147, 4286-4292).

Ainsi, le dibutyrate de phorbol (5ng/ml) et un Ca++ ionophore (0,5 µM) sont mis à incuber avec les thymocytes en absence ou en présence de différentes concentrations de CD336 (acide 4-((5,6,7,8-tetrahydro-5,5,8,8-tetramethyl-2-naphtalenyl)carboxamido) benzoïque).

Le tableau 3 suivant rassemble les résultats obtenus.

**Tableau 3**

| PRODUITS | QUANTITES (nM) | % de fragments d'ADN |
|---|---|---|
| P + C seul | - | 17 |
| CD336 seul | 30 | 0 |
| | 300 | 0 |
| | 3000 | 0 |
| (P + C) + CD336 | CD336 : 30 | 9,6 |
| | CD336 : 300 | 8,4 |
| | CD336 :3000 | 1,1 |
| P + C signifie dibutyrate de phorbol (5ng/ml) et un Ca++ ionophore (0,5 µM) | | |

Le pourcentage de fragment d'ADN dans ce tableau correspond à la différence de pourcentage de fragment d'ADN obtenu dans des thymocytes traitées et du pourcentage de fragment d'ADN obtenu dans des thymocytés non traitées (taux basal d'apoptose de ces thymocytes).

Ainsi, ce tableau montre clairement que l'apoptose induite par P + C est inhibée de manière dose-dépendante par le CD336 qui est un ligand agoniste spécifique des récepteurs de type RAR-α.

### EXEMPLE 4

Cette expérience montre l'efficacité in vivo de ligand antagoniste spécifique de RARγ en tant qu'inhibiteur d'apoptose, l'apoptose ayant été induite par un ligand agoniste spécifique de RARγ.

Des souris mâles NMRI de 4 semaines (vendus par la société LATI, Gôdôllo, Hongrie) ont été utilisées. Pour l'induction de l'apoptose dans le thymus, ces souris mâles ont été traitées par injection intraperitonéale unique avec 0,5 mg de l'acide 6-3-(1-Adamantyl)-4-hydroxyphényl)-2-naphtanoïque, dissout dans 40µl de DMSO et 0,5 ml d'éthanol à 20%. Pour voir l'effet inhibiteur d'apoptose induite par un ligand agoniste spécifique de RARγ dans le thymus, des souris mâles ont été traitées par injection intraperitonéale unique avec un mélange de 0,5 mg d'acide 6-3-(1-Adamantyl)-4-hydroxyphényl)-2-naphtanoïque (CD437) et de 5 mg d'acide 4-[7-(1-adamantyl)-6-méthoxyéthoxyméthoxy-2-naphtyl]benzoïque (CD2665), ce mélange ayant été dissout dans 40µl de DMSO et 0,5 ml d'éthanol à 20%. Pour le contrôle, des souris mâles ont été traitées par injection intraperitonéale unique d'un mélange de 40µl de DMSO et de 0,5 ml d'éthanol à 20%. Le CD2665 a également été testé seul dans les mêmes conditions que précdéemment à 0,5mg.

Le poids du thymus a été déterminé après 48 heures de traitement, les résultats sont rassemblés dans le tableau 4 suivant.

**Tableau 4**

| Traitement | poids du thymus (mg) | Etat général de l'animal |
|---|---|---|
| Contrôle | 98,2 ± 19,6 | normal |
| CD437 (0,5mg) | 31,3 ± 13,6 | normal |
| CD2665 (0,5mg) | 107,6 ± 21,7 | normal |
| CD437 (0,5mg) + CD2665 (5mg) | 63,3 ± 9,1 | normal |

Ainsi, on observe une involution du thymus après un traitement avec le CD 437 seul, aucun changement significatif n'est observé avec le CD2665, alors que l'association CD2665 et CD 437 présente une involution du thymus beaucoup moins importante que celle observée avec le CD 437 seul.

## Revendications

1. Utilisation d'au moins un ligand choisi parmi un ligand agoniste spécifique aux récepteurs de type RAR-α et un ligand antagoniste spécifique aux récepteurs de type RAR-γ dans la préparation d'une composition pharmaceutique destinée à diminuer le taux d'apoptose dans au moins une population cellulaire.

2. Utilisation selon la revendication précédente, **caractérisée en ce que** la population cellulaire correspond à des cellules dans lesquelles l'apoptose peut être régulée par induction et/ou inhibition par le biais des récepteurs de types RAR-γ et/ou RAR-α ou dans laquelle l'apoptose peut être régulée par induction par le biais des récepteurs des cellules T.

3. Utilisation selon l'une des revendications précédentes, **caractérisée en ce que** le ligand antagoniste spécifique aux récepteurs de type RAR-γ est choisi parmi l'acide 2-hydroxy-4-[7-(1-adamantyl)-6-benzyloxy-2-naphtyl]benzoïque, l'acide 2-hydroxy-4-[7-(1-adamantyl)-6-hexyloxy-2-naphtyl]benzoïque, l'acide 4-[7-(1-adamantyl)-6-méthoxyéthoxyméthoxy-2-naphtyl]benzoïque, l'acide 5-[7-(1-adamantyl)-6-benzyloxy-2-naphtyl]-2-thiophènecarboxylique, l'acide 4-[7-(1-adamantyl)-6-benzyloxy-2-naphtyl]benzoïque, l'acide 4-[7-(1-adamantyl)-6-benzyloxycarbonyl-2-naphtyl]benzoïque, l'acide 2-hydroxy-4-[7-(1-adamantyl)-6-(4-fluorobenzyl)oxy-2-naphtyl] benzoïque, l'acide 4-[7-(1-adamantyl)-6-heptyloxy-2-naphtyl]benzoïque, l'acide 6-[7-(1-adamantyl)-6-méthoxyéthoxyméthoxy-2-naphtyl]nicotinique, l'acide 2-hydroxy-4-[7-(1-adamantyl)-6-méthoxyéthoxyméthoxy-2-naphtyl]benzoïque, l'acide 2-chloro-4-[7-(1-adamantyl)-6-méthoxyéthoxyméthoxy-2-naphtyl]benzoïque, l'acide 4-[7-(1-adamantyl)-6-méthoxybutyloxy-2-naphtyl]benzoïque, l'acide 4-[7-(1-adamantyl)-6-méthoxyméthoxypropyl-2-naphtyl]benzoïque, l'acide 4-[7-(1-adamantyl)-6-méthoxyéthoxypropyl-2-naphtyl]benzoïque et l'acide 4-[7-(1-adamantyl)-6-acétyloxybutoxy-2-naphtyl]benzoïque.

4. Utilisation selon l'une des revendications précédentes, **caractérisée en ce que** le ligand antagoniste spécifique aux récepteurs de type RAR-γ présente une constante de dissociation de ce ligand pour les récepteurs de type RAR-γ au moins 20 fois inférieure à la constante de dissociation de ce ligand pour les récepteurs de type RAR-α, tel que l'acide 4-[7-(1-adamantyl)-6-méthoxyéthoxyméthoxy-2-naphtyl]benzoïque.

5. Utilisation selon la revendication 1 ou 2, **caractérisée en ce que** le ligand agoniste spécifique aux récepteurs de type RAR-α présente une constante de dissociation pour les récepteurs de type RAR-α au moins 20 fois inférieure à sa constante de dissociation pour les récepteurs de type RAR-γ.

6. Utilisation selon l'une des revendications 1, 2 et 5, **caractérisée en ce que** le ligand agoniste spécifique aux récepteurs de type RAR-α est choisi parmi l'acide 4-((5,6,7,8-tetrahydro-5,5,8,8-tetramethyl-2-naphtalenyl)carboxamido) benzoïque et l'acide 2-hydroxy-4-(5,5,8,8-tetramethyl-5,6,7,8-tetrahydro-naphtalene-2-carboxamido)-benzoique.

7. Utilisation selon l'une quelconque des revendications précédentes, **caractérisée en ce que** la composition pharmaceutique est destinée à traiter les maladies liées à un excès du taux d'apoptose.

8. Utilisation selon la revendication précédente, **caractérisée en ce que** les maladies liées à un excès du taux d'apoptose sont choisis parmi le syndrome d'immunodéficience du virus d'immunodéficience humain (H.I.V.), les maladies neuro-dégénératives, les syndromes myélodisplasiques, les syndromes d'ischémie, les maladies du foie induites par des toxines, l'alopécie, les dommages de la peau dus aux U.V., le lichen plan, l'atrophie de la peau, la cataracte ou encore le rejet de greffes.

9. Utilisation selon la revendication précédente, **caractérisée en ce que** les maladies neuro-dégénératives sont choisies parmi la maladie d'Azheimer, la maladie de Parkinson, la sclérose latérale amyotropique et d'autres maladies liées à une dégénération du cerveau, telle que la maladie de Creutzfeld Jakob.

## Claims

1. Use of at least one ligand which is selected from an agonist ligand which is specific for receptors of the RAR-α type and an antagonist ligand which is specific for receptors of the RAR-γ type in the preparation of a pharmaceutical composition which is intended for decreasing the rate of apoptosis in at least one cell population.

2. Use according to the preceding claim, **characterized in that** the cell population corresponds to cells in which apoptosis can be regulated by induction and/or inhibition using receptors of the RAR-γ and/or RAR-α type or in which apoptosis can be regulated by induction using T-cell receptors.

3. Use according to either of the preceding claims, **characterized in that** the antagonist ligand which is specific for receptors of the RAR-γ type is selected from 2-hydroxy-4-[7-(1-adamantyl)-6-benzyloxy-2-naphthyl]benzoic acid, 2-hydroxy-4-[7-(1-adamantyl)-6-hexyloxy-2-naphthyl]benzoic acid, 4-[7-(1-adamantyl)-6-methoxyethoxymethoxy-2-naphthyl]benzoic acid, 5-[7-(1-adamantyl)-6-benzyloxy-2-naphthyl]-2-thiophenecarboxylic acid, 4-[7-(1-adamantyl)-6-benzyloxy-2-naphthyl]benzoic acid, 4-[7-(1-adamantyl)-6-benzyloxycarbonyl-2-naphthyl]benzoic acid, 2-hydroxy-4-[7- (1-adamantyl) -6- (4-fluorobenzyl) oxy-2-naphthyl]benzoic acid, 4-[7-(1-adamantyl)-6-heptyloxy-2-naphthyl]benzoic acid, 6-[7-(1-adamantyl)-6-methoxyethoxymethoxy-2-naphthyl]nicotinic acid, 2-hydroxy-4-[7-(1-adamantyl)-6-methoxyethoxymethoxy-2-naphthyl]benzoic acid, 2-chloro-4-[7-(1-adamantyl)-6-methoxyethoxymethoxy-2-naphthyl]benzoic acid, 4-[7-(1-adamantyl)-6-methoxybutyloxy-2-naphthyl]benzoic acid, 4-[7-(1-adamantyl) -6-methoxymethoxypropyl-2-naphthyl]benzoic acid, 4-[7-(1-adamantyl)-6-methoxyethoxypropyl-2-naphthyl]benzoic acid and 4-[7-(1-adamantyl)-6-acetyloxybutoxy-2-naphthyl]benzoic acid.

4. Use according to one of the preceding claims, **characterized in that** the antagonist ligand which is specific for receptors of the RAR-γ type exhibits a dissociation constant of this ligand for receptors of the RAR-γ type which is at least 20 times lower than the dissociation constant of this ligand for receptors of the RAR-α type, such as 4-[7-(1-adamantyl)-6-methoxyethoxymethoxy-2-naphthyl]benzoic acid.

5. Use according to Claim 1 or 2, **characterized in that** the agonist ligand which is specific for receptors of the RAR-α type exhibits a dissociation constant for receptors of the RAR-α type which is at least 20 times lower than its dissociation constant for receptors of the RAR-γ type.

6. Use according to one of Claims 1, 2 and 5, **characterized in that** the agonist ligand which is specific for receptors of the RAR-α type is 4-((5,6,7,8-tetrahydro-5,5,8,8-tetramethyl-2-naphthalenyl)carboxamido)benzoic acid and 2-hydroxy-4- (5,5,8,8-tetramethyl-5,6,7,8-tetrahydronaphthalene-2-carboxamido)benzoic acid.

7. Use according to any one of the preceding claims, **characterized in that** the pharmaceutical composition is intended for treating diseases which are linked to an excessive rate of apoptosis.

8. Use according to the preceding claim, **characterized in that** the diseases which are linked to an excessive rate of apoptosis are selected from the human immunodeficiency virus (HIV) immunodeficiency syndrome, neurodegenerative diseases, myelodysplastic syndromes, ischaemic syndromes, liver diseases which are induced by toxins, alopecia, damage to the skin due to UV light, lichen planus, atrophy of the skin, cataract or else graft rejection.

9. Use according to the preceding claim, **characterized in that** the neurodegenerative diseases are selected from Alzheimer's disease, Parkinson's disease, amyotrophic lateral sclerosis and other diseases which are linked to degeneration of the brain, such as Creutzfeldt-Jakob disease.

## Patentansprüche

1. Verwendung mindestens eines Liganden, der unter den für die Rezeptoren vom RAR-α-Typ spezifischen, agonistisch wirkenden Liganden und den für die Rezeptoren vom RAR-γ-Typ spezifischen, antagonistisch wirkenden Liganden ausgewählt ist, bei der Herstellung einer pharmazeutischen Zusammensetzung, die dafür vorgesehen ist, das Ausmaß der Apoptose in mindestens einer Zellpopulation zu verringern.

2. Verwendung nach dem vorhergehenden Anspruch, **dadurch gekennzeichnet, daß** die Zellpopulation Zellen entspricht, in denen die Apoptose durch die Induktion und/oder Hemmung auf dem Umweg über die Rezeptoren vom RAR-γ-Typ und/oder RAR-α-Typ oder in der die Apoptose durch Induktion auf dem Umweg über die Rezeptoren von T-Zellen reguliert werden kann.

3. Verwendung nach einem der vorhergehenden Ansprüche, **dadurch gekennzeichnet, daß** der für die Rezeptoren vom RAR-γ-Typ spezifische, antagonistisch wirkende Ligand ausgewählt wird unter 2-Hydroxy-4-[7-(1-adamantyl)-6-benzyloxy-2-naphthyl]-benzoesäure, 2-Hydroxy-4-[7-(1-adamantyl)-6-hexyloxy-2-naphthyl]-benzoesäure, 4-[7-(1-Adamantyl)-6-methoxyethoxymethoxy-2-naphthyl]-benzoesäure, 5-[7-(1-Adamantyl)-6-benzyloxy-2-naphthyl]-2-thio-phencarbonsäure, 4-[7-(1-Adamantyl)-6-benzyloxy-2-naphthyl]-benzoesäure, 4-[7-(1-Adamantyl)-6-benzyloxycarbonyl-2-naphthyl]-benzoesäure, 2-Hydroxy-4-[7-1-(adamantyl)-6-(4-fluorbenzyl)-oxy-2-naphthyl]-benzoesäure, 4-[7-(1-Adamantyl)-6-heptyloxy-2-naphtyl]-benzoesäure, 6-[7-(1-Adamantyl)-6-methoxyethoxymethoxy-2-naph-thyl]-nicotinsäure, 2-Hydroxy-4-[7-(1-adamantyl)-6-methoxyethoxy-methoxy-2-naphthyl]-benzoesäure, 2-Chlor-4-[7-(1-adamantyl)-6-methoxyethoxymethoxy-2-naphthyl]-benzoesäure, 4-[7-(1-Adamantyl)-6-methoxybutyloxy-2-naphthyl]-benzoesäure, 4-[7-(1-Adamantyl)-6-methoxymethoxypropyl-2-naphthyl]-benzoesäure, 4-[7-(1-Adamantyl)-6-methoxyethoxypropyl-2-naphthyl]-benzoesäure und 4-[7-(1-Adamantyl)-6-acetyloxybutoxy-2-naphthyl]-benzoesäure.

4. Verwendung nach einem der vorhergehenden Ansprüche, **dadurch gekennzeichnet, daß** der für die Rezeptoren vom RAR-γ-Typ spezifische, antagonistisch wirkende Ligand eine Dissoziationskonstante dieses Liganden für die Rezeptoren vom RAR-γ-Typ aufweist, die mindestens 20mal kleiner ist als die Dissoziationskonstante dieses Liganden für die Rezeptoren vom RAR-α-Typ, wie z.B. 4-[7-(1-Adamantyl)-6-methoxyethoxymethoxy-2-naphthyl]-benzoesäure.

5. Verwendung nach Anspruch 1 oder 2, **dadurch gekennzeichnet, daß** der für die Rezeptoren vom RAR-α-Typ spezifische, agonistisch wirkende Ligand eine Dissoziationskonstante für die Rezeptoren vom RAR-α-Typ aufweist, die mindestens 20mal kleiner ist als seine Dissoziationskonstante für die Rezeptoren vom RAR-γ-Typ.

6. Verwendung nach einem der Ansprüche 1, 2 und 5, **dadurch gekennzeichnet, daß** der für die Rezeptoren vom RAR-α-Typ spezifische, agonistisch wirkende Ligand unter der 4-((5,6,7,8-Tetrahydro-5,5,8,8-tetramethyl-2-naphtalenyl)-carboxamido)-benzoesäure und der 2-Hydroxy-4-(5,5,8,8-tetramethyl-5,6,7,8-tetrahydronaphthalin-2-carboxamido)-benzoesäure ausgewählt wird.

7. Verwendung nach einem der vorhergehenden Ansprüche, **dadurch gekennzeichnet, daß** die pharmazeutische Zusammensetzung für die Behandlung der Krankheiten vorgesehen ist, die mit einer übermäßig starken Apoptose verbunden sind.

8. Verwendung nach dem vorhergehenden Anspruch, **dadurch gekennzeichnet, daß** die Krankheiten, die mit einer übermäßig starken Apoptose verbunden sind, unter dem durch das menschliche Immunschwäche-Virus (HIV) ausgelösten Immunschwächesyndrom, den neurodegenerativen Erkrankungen, den myelodisplastischen Syndromen, den Ischämie-Syndromen, den Erkrankungen der Leber, die durch Toxine ausgelöst werden, der Alopezie, den Schädigungen der Haut, die durch UV-Strahlung hervorgerufen werden, dem *Lichen planus*, der Atrophie der Haut, dem Katarakt und der Abstoßung von Transplantaten ausgewählt werden.

9. Verwendung nach dem vorhergehenden Anspruch, **dadurch gekennzeichnet, daß** die neurodegenerativen Erkrankungen unter der Alzheimer-Krankheit, der Parkinson-Krankheit, der amyotropen lateralen Sklerose und anderen Erkrankungen, die mit einer Zerstörung des Gehirns, wie der Creutzfeld-Jakob-Krankheit, ausgewählt werden.
